(19) Europäisches Patentamt
European Patent Office
Office européen des brevets

(11) Publication number: 0 034 650
A1

(12) # EUROPEAN PATENT APPLICATION

(21) Application number: 80106316.5

(22) Date of filing: 16.10.80

(51) Int. Cl.$^3$: C 07 C 41/03
B 01 J 23/02, B 01 J 31/00

(30) Priority: 13.02.80 US 121072

(43) Date of publication of application: 02.09.81 Bulletin 81/35

(84) Designated Contracting States: BE DE FR GB SE

(71) Applicant: CONOCO INC.
1000 South Pine Street P.O. Box 1267
Ponca City Oklahoma 74601(US)

(72) Inventor: Kang, Yang
2501 Robin Road
Ponca City, Oklahoma 74601(US)

(72) Inventor: Washecheck, Paul Howard
2801 Canterbury
Ponca City, Oklahoma 74601(US)

(72) Inventor: Nield, Gerald Lee
837 Edgewood
Ponca City, Oklahoma, 74601(US)

(74) Representative: Patentanwälte Dipl.-Ing. A. Grünecker, Dr.-Ing. H. Kinkeldey, Dr.-Ing. W. Stockmair, Dr. rer. nat. K. Schumann, Dipl.-Ing. P.H. Jakob, Dr. rer. nat. G. Bezold Maximilianstrasse 43
D-8000 München 22(DE)

(54) **A method for alkoxylation of alkanols.**

(57) Barium-containing alkoxylation systems are used as catalysts for the alkoxylation of alcohols of all classes. The reaction is carried out at temperatures of from about 90 to about 260°C to yield an alkoxylation product. The products obtained have very narrow adduct distribution with low levels of by-products and unreacted free alcohols. Several classes of materials which promote these reactions are disclosed.

EP 0 034 650 A1

# A METHOD FOR ALKOXYLATION OF ALKANOLS

This invention relates to the production of alkoxylated alcohols by reacting said alcohols in the presence of barium-containing catalysts and promoters. More particularly, this invention relates to the production of alkoxylated alcohols by reacting said alcohols in the presence of these barium-containing catalysts with adducting materials such as ethylene oxide and propylene oxide.

The general reaction of alcohols and adducting materials such as ethylene oxide to form alkoxylated alcohols (ethylene oxide adducts) has long been known and practiced on a commercial scale. For example, ethylene oxide adducts have been used as detergents and cleaning agents, domestic and industrial laundry detergents, detergent builders, polishers, sanitizers, and dry-cleaning materials. Other users include the pulp and paper industry and the fiber industry. These materials are especially adapted to these uses since they have functional properties such as wetting power, foaming, emulsifying and dispersing abilities as well as solubilization and detergent abilities to facilitate their use.

Much literature is available in the general area of ethoxylation of alcohols. Many references are also available relating to the catalytic ability of various materials and the mechanism and kinetics of these reactions. For example, French Patent 1,365,945 teaches the use of compounds containing an active hydrogen atom reacted with ethylene oxide in the presence of an alkali metal base.

Acidic catalysts in general are also known. However, the ethoxylation of alcohols invariably produces a distribution of various adducts. For example on surfactant applications, an adduct of too few ethylene oxide molecules is not effective because of poor solubility. In contrast, an adduct with too many ethylene oxide molecules is likewise undesirable because of surface tension reduction per unit mass decreases drastically with increase in the molecular weight. Thus it has long been essential to produce and use ethoxylates with as sharp a distribution in the desired mole adduct range (3 to 10 usually) as

possible. Acid-catalyzed reactions produce such alkoxylates but these catalysts produce higher levels of some harmful side products (such as dioxane) which must be separated and removed prior to use.

Russian Patent 523,074 teaches that alkali metals and various carbonates can be used to catalyze these reactions. The side product formation in the base-catalyzed reactions is very low, but in base-catalyzed reactions the adduct distribution is undesirably broad. The result is that a large proportion of the product obtained is not useful or is less desirable because of distribution.

Representative of but not exhaustive of the art in this area is U.S. Patent 3,328,467 which describes the use of zeolites and modified zeolites as catalysts in ethoxylation reactions. French Patent 1,557,407 uses triethyl oxonium fluoroborate to catalyze such reactions. Indeed, the art abounds with references to alkali metal hydroxides such as sodium and potassium hydroxides, tertiary amines and sodium metal. German Offenlegungsschrift 2,639,564 teaches polyalkoxylation of active hydrogen compounds in the presence of a sodium fluoroborate or perchlorates of metal such as magnesium, calcium, manganese, or zinc. U.S. Patent 3,969,417 uses tertiary oxonium salts as a catalyst.

U.S. Patent 3,830,850 describes adding sodium, potassium, lithium, rubidium, cesium, calcium, barium, or strontium to condense phenols with formaldehyde, then adding ethylene oxide to the condensation product in an ethoxylation reaction. However, all these materials have the disadvantages described and set forth above.

Great benefit would be provided by a catalyst system which provides the low by-product levels of base catalysts yet has the narrow distribution of the preferred mole adducts obtained from acid catalysts. Such a catalyst which would promote the narrowing of the product distribution curve would contribute significantly to the intrinsic value of the ethoxylate produced. Such a catalyst is described in U.S. application Serial Number 916,421, filed June 6, 1978. However, this catalyst has an induction period ranging up to about

BAD ORIGINAL

20 minutes at 178°C and produces from 1 to 2% polyethylene glycol in the product which while small, is still undesirably large.

It is therefore an object of the present invention to provide a catalyst system which will yield a narrow mole adduct distribution from the reaction of alcohols of all classes with materials such as ethylene oxide and propylene oxide while providing low levels of undesirable by-products and unreacted free alcohols, yet provide a reaction which is immediately effective with reduced induction period. Other objects will become apparent to those skilled in this art as the description proceeds.

It has now been discovered according to the present invention that alkoxylation of alkanols containing from about 4 to about 36 carbon atoms can be carried out by contacting the alcohols with an alkoxylation agent in the presence of at least one material selected from the group consisting of barium hydride, barium oxide, barium hydroxide, hydrated barium hydroxide, or mixtures of these together with an effective amount of a catalyst promoter or mixture of catalyst promoters, wherein the alkoxylation reaction is carried out at temperatures of from about 120°C to about 260°C and wherein the catalyst promoter is selected from the group consisting of

a) polyols having a boiling point above 100°C and containing a total of 2 to 30 carbon atoms; and having 2 or more hydroxyl containing groups of the general formula

$$R_1 - \overset{\displaystyle R_2}{\underset{\displaystyle R_3}{\overset{|}{\underset{|}{C}}}} - OH$$

wherein $R_1$, $R_2$, and $R_3$ are, independently, linear or branched acyclic groups, alicyclic groups, aryl groups, cyclic groups, or hydrogen and wherein the R-designated groups can in addition contain one or more functional groups selected from the group consisting of ether, amine, carboxyl, halogen, carboxyl, nitro or amide;

BAD ORIGINAL

b) aldehydes and ketones having boiling points above 100°C and containing a total of from 2 to 30 carbon atoms, and having one or more carbonyl containing groups of the general formula

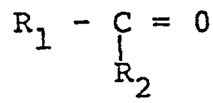

wherein $R_1$ and $R_2$ are, independently, hydrogen, linear or branched acyclic groups, alicyclic groups, cyclic groups, or aryl groups and wherein the R-designated groups can in addition contain one or more functionalities selected from the group consisting of carboxyl, hydroxyl, ether, halogen, nitro, amine, or amide;

c) primary, secondary or tertiary amides having a boiling point of above 100°C and containing a total of from 1 to 30 carbon atoms and containing 1 or more amide containing groups of the general formula

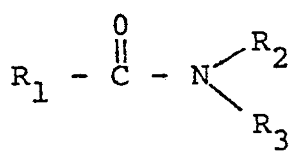

wherein $R_1$, $R_2$, and $R_3$ are, independently hydrogen, linear or branched acyclic groups, alicyclic groups, cyclic groups or aryl groups and wherein the R-designated groups can in addition contain one or more other functionalities selected from the group consisting of hydroxyl, ether, carboxyl, carbonyl, amine, nitro, or halogen;

d) primary, secondary or tertiary amines having a boiling point above 100°C, containing from a total of 1 to 30 carbon atoms and containing 1 or more amine containing groups of the general formula

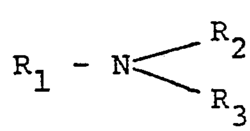

wherein $R_1$, $R_2$ and $R_3$ are, independently, hydrogen, linear or branched acyclic groups, alicyclic groups, cyclic groups, or aryl groups, and wherein

BAD ORIGINAL

the R-designated groups can in addition contain one or more functionalities selected from the group consisting of hydroxyl, ether, carbonyl, halogen, carboxyl, nitro or amide;

e) organic acids having a boiling point of above 100°C, containing from a total of 1 to 30 carbon atoms and having 1 or more carboxylic acid containing groups of the general formula

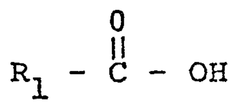

wherein $R_1$ is hydrogen, a linear or branched acyclic group, alicyclic group, cyclic group, or aryl group and wherein the R group can in addition contain one or more functionalities selected from the group consisting of carbonyl, hydroxyl, halogen, ether, nitro, amine, or amide;

f) phenols having a boiling point of above 100°C, containing a total of from 6 to 30 carbon atoms and having 1 or more functionalities of the general formula

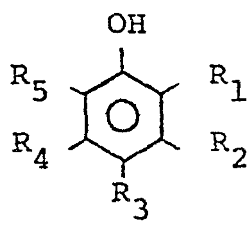

wherein $R_1$, $R_2$, $R_3$, $R_4$, and $R_5$ are, independently hydrogen, halogen, hydroxyl, nitro, ether, or carbonyl, linear or branched acyclic groups, alicyclic groups, cyclic groups, aryl groups, or substituted aryl groups and wherein in addition the R-designated groups can contain one or more functionalities selected from the group consisting of halogen, ether, nitro, carboxyl, carbonyl, amine, amide, or hydroxyl. Boiling points are measured at atmospheric pressure, and may be proportionally higher or lower as pressure is altered.

Barium hydride is an effective catalyst whether used alone or in combination with a phenol or a substitute

BAD ORIGINAL

phenol or the formula described in (f), where substitutions are, independently, hydrogen or alkyl groups containing from 1 to 10 carbon atoms.

In our co-pending application SN 36,273, filed May 4, 1979, we described phenolic promoters having alkyl groups containing from 1 to 10 carbon atoms. In the present invention we have discovered that such acyclic and alicyclic groups containing at least 11 carbon atoms can also be used, in addition to or in combination with other functional groups described.

Representative examples of various polyol promoters effective in the process of the instant invention are:

ethylene glycol
1,2-propylene glycol
1,4-butanediol
1,6-hexanediol
1,10-decanediol
1,3-butylene glycol
diethylene glycol
diethylene glycol monobutyl ether
diethylene glycol monomethyl ether
diethyl glycol monoethyl ether
dipropylene glycol
dipropylene glycol monomethyl ether
ethylene glycol monomethyl ether
ethylene glycol monoethyl ether
ethylene glycol monobutyl ether
hexylene glycol
mannitol
sorbitol
pentaerythritol
dipentaerythritol
tripentaerythritol
trimethylolpropane
trimethylolethane
neopentyl glycol
diethanolamine
triethanolamine
diisopropanolamine
triisopropanolamine
1,4-dimethylolcyclohexane
2,2-bis(hydroxymethyl)propionic acid
1,2-bis(hydroxymethyl)benzene
4,5-bis(hydroxymethyl)furfural
4,8-bis(hydroxymethyl)tricyclo[5,2,1,0] decane
tartaric acid
2-ethyl-1,3-hexanediol
2-amino-2-ethyl-1,3-propanediol
triethylene glycol
tetraethylene glycol
glycerol
ascorbic acid

BAD ORIGINAL

Representative examples of various aldehydes and ketones effective in the process of the present invention are:

lauryl aldehyde
benzaldehyde
2-undecanone
acetophenone
2,4-pentandione
acetylsalicylic acid
ortho-chlorobenzaldehyde
para-chlorobenzaldehyde
cinnamic aldehyde
diisobutyl ketone
ethylacetoacetate
ethyl amyl ketone
camphor
para-hydroxybenzaldehyde
2-carboxybenzaldehyde
4-carboxybenzaldehyde
salicylaldehyde
octyl aldehyde
decyl aldehyde
p-methoxybenzaldehyde
p-aminobenzaldehyde
phenylacetaldehyde
acetoacetic acid
2,5-dimethoxybenzaldehyde
1-naphthyl aldehyde
terephthaldehyde

Representative examples of amides which are effective promoters in the process of the instant invention are:

formamide
benzamide
acetanilide
salicylamide
acetoacetanilide
ortho-acetoacetotoluidide
acrylamide
N,N-diethyltoluamide
N,N-dimethylacetamide
N,N-dimethylformamide
phthalimide
octylamide
decylamide
laurylamide
stearylamide
N,N-dimethylollaurylamide
N,N-dimethylacrylamide
para-chlorobenzamide

para-methoxybenzamide
para-aminobenzamide
para-hydroxybenzamide
ortho-nitrobenzamide
N-acetyl-para-aminophenol
2-chloroacetamide
oxamide
N,N-methylene-bis-acrylamide

Representative examples of amines which are effective promoters in the process of the present invention are:

aniline
benzylamine
hexadecylamine
triphenylamine
aminoacetic acid
anthranilic acid
cyclohexylamine
tert-octylamine
ortho-phenylenediamine
meta-phenylenediamine
para-phenylenediamine
N-acetyl-para-aminophenol
2-amino-4-chlorophenol
2-amino-2-ethyl-1,3-propanediol
ortho-aminophenol
para-aminophenol
para-aminosalicylic acid
benzyl-N,N-dimethylamine
tert-butylamine
2-chloro-4-aminotoluene
6-chloro-2-aminotoluene
meta-chloroaniline
ortho-chloroaniline
para-chloroaniline
4-chloro-2-nitroaniline
cyclohexylamine
dibutylamine
2,5-dichloroaniline
3,4-dichloroaniline
dicyclohexylamine
diethanolamine
N,N-diethylethanolamine
N,N-diethyl-meta-toluidine
N,N-diethylaniline
diethylenetriamine
diisopropanolamine
N,N-dimethylethanolamine
N,N-dimethylaniline
2,4-dinitroaniline
diphenylamine
ethyl-para-aminobenzoate
N-ethylethanolamine

BAD ORIGINAL

N-e nyl-1-naphthylamine
N-ethyl-ortho-toluidine
N-ethylaniline
ethylenediamine
hexamethylenetetraamine
2,4-lutidine
N-methylaniline
methyl anthranilate
p,p'-diaminodiphenyl methane
ortho-nitroaniline
para-nitroaniline
tert-octylamine
piperazine
ethanolamine
isopropanolamine
ortho-toluidine
para-toluidine
2,4-tolyenediamine
triethanolamine
tributylamine
triisopropanolamine
2,4-dimethylxylidine
para-methoxyaniline
nitrilotriacetic acid
N-phenyl-1-naphthylamine

Representative examples of acids which are effective promoters in the process of the present invention are:

formic acid
acetic acid
valeric acid
heptanoic acid
2-ethylhexanoic acid
lauric acid
stearic acid
oleic acid
tall oil acids
hydrogenated tall oil acids
benzoic acid
salicylic acid
adipic acid
azelaic acid
fumaric acid
citric acid
acrylic acid
aminoacetic acid
para-aminosalicylic acid
anthranilic acid
butyric acid
propionic acid
ricinoleic acid
chloroacetic acid
ortho-chlorobenzoic acid
2,4-dichlorophenoxyacetic acid

tert-decanoic acid
para-aminobenzoic acid
abietic acid
itaconic acid
lactic acid
glycolic acid
malic acid
maleic acid
cinnamic acid
para-hydroxybenzoic acid
methacrylic acid
oxalic acid
myristic acid
palmitic acid
tert-pentanoic acid
pherylacetic acid
mandelic acid
sebacic acid
tallow fatty acids
hydrogenated tallow fatty acids
tartaric acid
trichloroacetic acid
2,4,5-trichlorophenoxyacetic acid
undecylenic acid
crotonic acid
pelargonic acid
acetoacetic acid
para-nitrobenzoic acid
ascorbic acid
nitrilotracetic acid
naphthenic acids
1-naphthoic acid
trimellitic acid

Representative examples of various phenols which are promoters for barium-containing catalysts of the present invention are:

phenol
ortho-cresol
meta-cresol
para-cresol
2,4-dimethylphenol
2,5-dimethylphenol
2,6-dimethylphenol
ortho-chlorophenol
meta-chlorophenol
para-chlorophenol
para-nitrophenol
para-methoxyphenol
salicylic acid
meta-hydroxyacetophenone
para-aminophenol
ortho-phenylphenol
nonylphenol
octylphenol
t-butyl-para-cresol
hydroquinone

catechol
resorcinol
pyrogallol
1-naphthol
2-naphthol
4,4'-isopropylidenediphenol (bisphenol A)
methyl salicylate
benzyl salicylate
4-chloro-2-nitrophenol
para-t-butylphenol
2,4-di-t-amylphenol
2,4-dinitrophenol
para-hydroxybenzoic acid
8-hydroxyquinoline
methyl para-hydroxybenzoate
2-nitro-para-cresol
ortho-nitrophenol
para-phenylphenol
phenyl salicylate
salicylaldehyde
p-hydroxy benzaldehyde
2-amino-4-chlorophenol
ortho-aminophenol
salicylamide
2,4-dichlorophenol
2,5-dichlorophenol
2,5-dichlorohydroquinone

Certain alcohols can also act as promoters for ethoxylation of alcohols using barium-containing catalysts. Such alcohols are more acidic than normal alcohols such that the association constant is greater (or the pKa is lower) than normal alcohols. Thus, these alcohol promoters would have a pKa less than 17.

These alcohols are those having the general formula

$$R_1 - \underset{R_3}{\overset{R_2}{C}} - OH$$

wherein $R_1$, $R_2$ and $R_3$ are, independently, hydrogen, linear or branched acyclic groups, alicyclic groups, aryl groups, or cyclic groups and wherein the R-designated groups can in addition contain functional groups selected from the group consisting of halogen, nitro, carboxyl, amine, carbonyl, ether, and amide. The molecule can contain a total of from 2 to 30 carbon atoms.

Representative examples of these effective alcohol promoters are:

triphenylmethanol
trichloroethanol
trifluoroethanol
2-nitroethanol
2-chloroethanol
2,2-dichloroethanol
2-methoxyethanol
2-chlorocyclohexanol
ortho-chlorobenzyl alcohol

Thus, the instant invention describes a method for the alkoxylation of alcohols comprising contacting said alcohols with an alkoxylating agent in the presence of barium-containing catalysts together with an effective amount of a polyol, aldehyde, ketone, amine, amide, carboxylic acid, substituted phenol or alcohol or mixtures of these to promote the reaction. Normally, the alkoxylation agent will be ethylene oxide or propylene oxide. The instant invention can be carried out at temperatures of from about 90°C to about 260°C. Normally, the alcohols reactive under the process of the instant invention will contain from about 4 to about 36 carbon atoms but alcohols of from about 4 to about 24 carbon atoms are more common. Those alcohols containing from about 8 to about 18 carbon atoms are most used for commercial processes.

The process of the instant invention can be carried out at ambient pressures. However, pressures of up to 100 pounds per square inch gauge (psig) can also be used. Pressures below about 60 psig are preferred. In addition, pressures below ambient can be used. It is clear that while pressure or lack of pressure is not a detriment to the process of the instant invention, it is simply more convenient to carry out the reaction in the pressure range of from about atmospheric to about 100 psig.

The instant invention is normally carried out at temperatures of from about 120 to about 260°C. However, for practical purposes, commercial operations will normally be carried out in the temperature range of from about 150 to about 200°C. Temperatures in the range of from 160°C to about 190°C are most preferred.

BAD ORIGINAL

Reactions can be carried out in the presence of any alkoxylation agent which produces a mole adduct of the sort desired. Normally such agents are alpha or beta alkylene oxides. In most commercial operations, either ethylene oxide, propylene oxide, or mixtures of these will be used to produce an adduct. Of these products, ethylene oxide is most preferred.

Reaction products can have any desired content of alkoxylating agent such as ethylene oxide, but will normally range from about 30 to about 80% content of ethylene oxide (EO) based on weight. However, for most purposes, the content of ethylene oxide will range from about 40% to about 70% by weight. The amount of EO present in the reaction is not critical other than the minimum amount necessary to provide sufficient units to reach the mole adduct level desired for the alcohol being reacted.

The barium-containing catalyst of the present invention is a basic catalyst which provides a sharp distribution as to the mole adducts formed, while greatly reducing the amount of unreacted free alcohols and undesirable by-products normally found in sharp distribution reactions. The instant invention adds to this barium catalyst an effective amount of phenol, acid, amine, aldehyde, polyol, ketone, amide or alcohol in order to further reduce by-product reactions and to reduce or eliminate the induction period necessary for alkoxylation to begin.

Representative examples of barium-containing catalysts are barium metal, $BaH_2$, BaO, $Ba(OH)_2$ and $Ba(OH)_2 \cdot X\ H_2O$ where X represents the number of water molecules present. These barium compounds alone are active in the process of the instant invention and are extremely active when used with an effective amount of a co-catalyst or promoter.

When used, these catalyst mixtures can be used in any desired quantity. The larger the quantity used, the more quickly the reaction goes to completion, although larger quantities do not appear to significantly alter the distribution obtained. However, for practical purposes, normally at least about 0.1 weight percent barium catalyst, based upon the weight of the alcohol to be reacted, would be

BAD ORIGINAL

present in the reaction. However, from 0.1 to about 5.0 weight percent is more commonly used and 0.1 to 2.0 weight percent is preferred. The amount of promoter or co-catalyst which should be present with the barium catalyst is generally an effective amount. The effect of the co-catalyst or promoter becomes significant at about 0.1% by weight based upon the weight of the alcohol to be reacted. It is logical to expect an upper limit after which the amount of promoter present will produce no additional benefits.

Normally, these materials will be added to the barium catalysts in amounts ranging from about 0.1 to about 2% by weight based upon the weight of the alcohol. Although amounts ranging from about 0.15 to about 1.5 are preferred, and amounts ranging from about 0.3 to about 0.8% by weight based upon the weight to be reacted is most preferred. However it is very apparent that these limits can be varied substantially.

While the instant invention is effective with all classes of alkanols, including primary, secondary, tertiary, linear and branched, linear and branched primary alkanols are the most commonly used alcohols and are the preferred alcohols of the instant invention. Representative examples of such alcohols are those derived by hydrogenation of natural fats and oils, such as CO and TA alcohols, trademark of and sold by Proctor and Gamble Co., such as CO 1214N alcohol, CO 1618 alcohol, and TA 1618 alcohol, and ADOL alcohols, trademark of and sold by Ashland Oil Co., such as ADOL 54 alcohol, ADOL 61 alcohol, ADOL 64 alcohol, ADOL 60 alcohol and ADOL 66 alcohol. Alcohols produced by Ziegler chemistry can also be ethoxylated. Examples of these alcohols are ALFOL alcohols, trademark of and sold by Conoco Inc, such as ALFOL 1012 alcohol, ALFOL 1214 alcohol, ALFOL 1412 alcohol, ALFOL 1618 alcohol, ALFOL 1620 alcohol; and EPAL alcohols, trademark of and sold by Ethyl Chemical Co., such as EPAL 1012 alcohol, EPAL 1214 alcohol, EPAL 1418 alcohol. The invention is extremely useful for oxo alcohols (hydroformylation) produced from olefins. Examples of such alcohols are NEODOL alcohol, trademark of and sold by Shell Oil Co., such as NEODOL 23 alcohol, NEODOL 25 alcohol, NEODOL 45 alcohol; TERGITOL-L, trademark of Union Carbide Corp., such as

TERGITOL-L 125 alcohol; LIAL alcohols, trademark of and sold by Liquichimica Co. such as LIAL 125; and isodecyl and tri decyl alcohols, sold by Exxon Corp., such as isodecyl alcohol and tridecyl alcohol. Guerbet alcohols can also be ethoxylated. Representative examples of these alcohols are STANDAMUL alcohols trademark of and sold by Henkel Chemical Co., such as STANDAMUL GT-12 alcohol, STANDAMUL GT-16 alcohol, STANDAMUL GT-20 alcohol, STANDAMUL GT-1620 alcohol. Secondary alcohols can also be used, such as TERGITOL 15 alcohol, trademark of and sold by Union Carbide Corp.

Representative examples of such alcohols are 1-decanol; 1-undecanol; 1-dodecanol; 1-tridecanol; 1-tetra-decanol; 1-pentadecanol; 1-hexadecanol; 1-heptadecanol; 1-octadecanol; 1-nonadecanol; 1-eicosanol; 1-docosanol; 2-methyl-1-undecanol; 2-propyl-1-nonanol; 2-butyl-1-octanol; 2-methyl-1-tridecanol; 2-ethyl-1-dodecanol; 2-propyl-1-undecanol; 2-butyl-1-decanol; 2-pentyl-1-nonanol; 2-hexyl-1-octanol; 2-methyl-1-pentadecanol; 2-ethyl-1-tetradecanol; 2-propyl-1-tridecanol; 2-butyl-1-dodecanol; 2-pentyl-1-undecanol; 2-hexyl-1-decanol; 2-heptyl-1-decanol; 2-hexyl-1-nonanol; 2-octyl-1-octanol; 2-methyl-1-heptadecanol; 2-ethyl-1-hexadecanol; 2-propyl-1-pentadecanol; 2-butyl-1-tetradecanol; 1-pentyl-1-tridecanol; 2-hexyl-1-dodecanol; 2-octyl-1-decanol; 2-nonyl-1-nonanol; 2-dodecanol; 3-dodecanol; 4-dodecanol; 5-dodecanol; 6-dodecanol; 2-tetra-decanol; 3-tetradecanol; 4-tetradecanol; 5-tetradecanol; 6-tetradecanol; 7-tetradecanol; 2-hexadecanol; 3-hexadecanol; 4-hexadecanol; 5-hexadecanol; 6-hexadecanol; 7-hexadecanol; 8-hexadecanol; 2-octadecanol; 3-octadecanol; 4-octadecanol; 5-octadecanol; 6-octadecanol; 7-octadecanol; 8-octadecanol; 9-octadecanol; 9-octadecenol-1; 2,4,6-tri-methyl-1-heptanol; 2,4,6,8-tetramethyl-1-nonanol; 3,5,5-trimethyl-1-hexanol; 3,5,5,7,7-pentamethyl-1-octanol; 3-butyl-1-nonanol; 3-butyl-1-undecanol; 3-hexyl-1-undecanol; 3-hexyl-1-tridecanol; 3-octyl-1-tridecanol; 2-methyl-2-undecanol; 3-methyl-3-undecanol; 4-methyl-4-undecanol; 2-methyl-2-tridecanol; 3-methyl-3-tridecanol; 4-methyl-3-tridecanol; 4-methyl-4-tridecanol; 3-ethyl-3-decanol; 3-ethyl-3-dodecanol; 2,4,6,8-tetramethyl-2-nonanol; 2-methyl-3-undecanol; 2-methyl-4-undecanol; 4-methyl-2-undecanol; 5-methyl-2-undecanol; 4-ethyl-2-decanol; 4-ethyl-3-decanol:

tetracosanol; hexacosanol; octacosanol; triacontanol; dotriacontanol; hexatriacontanol; 2-decyltetradecanol; 2-dodecylhexadecanol; 2-tetradecyloctadecanol; 2-hexadecyl-eicosanol.

Generally, the treatment of alcohols with ethylene oxide yield a non-ionic detergent since hydrogen bonding to the numerous oxygen atoms makes the polyether end of the molecule water soluble. Alternatively, the ethoxylates can be converted into sulfates and used in the form of alkali metal or ammonium salts.

The instant invention provides for the production of highly efficient alcohol alkoxylates from primary, secondary and tertiary branched chain and straight chained alcohols in a novel highly unexpected manner. Of these, primary alkanols are preferred. These alkanols normally have from about 4 to about 20 carbon atoms, but from about 4 to about 36 carbon atoms can also be used. Reaction products are useful as non-ionic surface active agents with high wetting power and are composed with mixtures of monoalkyl ethers of polyethylene glycol.

Thus in the preferred form of the instant invention, ethylene oxide is reacted with a branched chain or straight chain higher alkanol in the presence of barium oxide, barium hydroxide, or other barium bases promoted by an effective amount of amines, amides, aldehydes, polyols, carboxylic acids, ketones, alcohols and substituted phenols.

The invention is more concretely described with reference to the examples below wherein all parts and percentages are by weight unless otherwise specified. The examples are provided to illustrate the instant invention and not to limit it.

Example 1

All examples were carried out in a stainless steel reactor (having a magnetic stirrer). This stainless steel reactor having the capacity of 600 cubic centimeters (cc) was charged with 120 grams of Neodol 25 alcohol (trademark of and sold by Shell Chemical Co., a product of an hydro-formylation reaction). $Ba(OH)_2 \cdot H_2O$ in an amount of 0.2 grams and 0.3 grams of 2-naphthol co-catalyst or promoter

was added. After purging with nitrogen at 250cc per minute for one hour at 150°C, the reactor was evacuated and the temperature raised to about 175°C. Ethylene oxide was then introduced to a total pressure of about 40 pounds per square inch gauge (psig) and ethylene oxide (EO) uptake of 180 grams was allowed to proceed at this pressure. After ethoxylation the catalyst was neutralized. The improved reaction rate achieved by the use of 2-naphthol as a promoter is shown in Figure 1, which directly compares the reaction rates of the two materials. The desired ethylene oxide distribution peak is shown in Figure 2. The reaction product was analyzed and found to contain a free alcohol content of 1.9%.

Example 2

As a comparative example to Example 1, the Example 1 was repeated exactly except that no 2-naphthol was added as a promoter. Reaction time is set forth in Figure 1 at a comparative basis. An examination of the figure will clearly show the improved reaction rate to example 1 as compared to the present Example 2.

Example 3

An experiment was carried out exactly as described in Example 1 except that 0.3 grams of 2-ethyl-hexanoic acid was used as a catalyst promoter. The ethoxylation time was reduced from 185 minutes to 135 minutes because of the effectiveness of the promoter. The rate of ethoxylation is set forth in Figure 3.

Example 4

An experiment was carried out exactly as described in Example 1 except that 0.27 grams of heptanoic acid was used. The ethoxylation time was reduced from 185 minutes to 150 minutes as a result of using heptanoic acid promoter. The reaction product was comparable in quality to that obtained using barium oxide alone.

Example 5

A 600 cubic centimeter (cc) stainless steel reactor is charged with 120 grams of Alfol 1214 alcohol

(Trademark of and sold by Conoco Inc) and a catalyst. The catalyst is $BaH_2$ (0.5 grams). After purging the reactor with nitrogen at the rate of 500 cc's per minute for 30 minutes at 150°C, the reactor is evacuated and the temperature raised to 175°C. Ethylene oxide (EO) is introduced to a total pressure of 40 pounds per square inch gauge (psig) and EO uptake at this constant pressure is measured as a function of time. After ethoxylation, the base is neutralized. The experiment will show that barium hydride is an extremely active catalyst.

The catalysts and method of the present invention are extremely well suited for the alkoxylation of alcohols produced by olefins by hydroformylation (or oxo/hydrogenation). Such alcohols have, in the past, presented difficulty when used as reactants for alkoxylation and particularly ethoxylation because of the high concentration of unreacted alcohols. The catalysts of the present invention produce extremely good ethoxylates using these alcohols.

It is apparent that the instant invention provides a method for obtaining high mole adduct alkoxylates of alcohols having a very narrow, highly desirable distribution range. In addition, the products have low amounts of by-products and unreacted free alcohols together with a desirably fast reaction rate. Promoters of the present invention greatly reduce the previously noted induction period present with these catalysts.

Although exemplified as a batch reaction, the catalysts and promoters of the present invention are extremely well suited to continuous reaction methods. The reaction products are of extremely high quality and quantity.

While certain embodiments and details have been shown for the purpose of illustrating this invention, it will be apparent to those skilled in this art that various changes and modifications may be made herein without departing from the spirit or scope of the invention.

We claim:

1. A method for the alkoxylation of alkanols containing from about 2 to about 36 carbon atoms, comprising carrying out said alkoxylation in the presence of basic barium-containing catalyst together with an effective amount of a catalyst promoter or mixture of catalyst promoters, where the alkoxylation reaction is carried out at temperatures of from about 120°C to about 260°C and wherein the catalyst promoter is selected from the group consisting of

a) polyols containing a total of from 2 to 30 carbon atoms and having a boiling point above 100°C;
b) aldehydes and ketones containing a total of from 2 to 30 carbon atoms and having a boiling point above 100°C;
c) amides containing a total of from 1 to 30 carbon atoms and having a boiling point above 100°C;
d) amines containing a total of from 1 to 30 carbon atoms and having a boiling point above 100°C;
e) organic acids containing a total of from 1 to 30 carbon atoms and having a boiling point of 100°C;
f) phenols containing a total of from 6 to 30 carbon atoms and having a boiling point above 100°C.

2. A method as described in claim 1 wherein

a) the polyol promoters contain 2 or more hydroxyl containing groups of the general formula

$$R_1 - \overset{\displaystyle R_2}{\underset{\displaystyle R_3}{\overset{|}{\underset{|}{C}}}} - OH$$

wherein $R_1$, $R_2$, and $R_3$ are, independently, linear or branched acyclic groups, alicyclic groups, cyclic groups, aryl groups or hydrogen and wherein the R-designated groups in addition can contain one or more functional groups selected from the group consisting of ether, amine, carboxyl, halogen, nitro, carbonyl, and amide;

b) the aldehyde and ketone promoters contain one or more carbonyl containing groups of the general formula

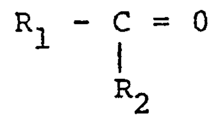

BAD ORIGINAL

wherein $R_1$, and $R_2$ are, independently, linear or branched acyclic groups, alicyclic groups, cyclic groups, aryl groups, or hydrogen, and wherein the R-designated groups in addition can contain one or more functional groups selected from the group consisting of hydroxyl, ether, carboxyl, carbonyl, amine, nitro or halogen;

c) the primary, secondary or tertiary amide promoters contain one or more amide containing groups of the general formula

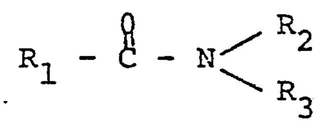

wherein $R_1$, $R_2$, and $R_3$ are, independently, hydrogen, linear or branched acyclic groups, alicyclic groups, cyclic groups or aryl groups and wherein the R-designated groups can in addition contain one or more functional groups selected from the group consisting of hydroxyl, ether, carboxyl, carbonyl, amine, nitro or halogen;

d) the primary, secondary or tertiary amine promoters contain one or more amine containing groups of the general formula

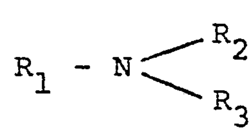

wherein $R_1$, $R_2$, and $R_3$ are, independently, hydrogen, linear or branched acyclic groups, alicyclic groups, cyclic groups, or aryl groups, and wherein the R-designated groups can in addition contain one or more functional groups selected from the group consisting of hydroxyl, ether, carbonyl, halogen, carboxyl, nitro, or amide;

e) the organic acid promoters contain one or more carboxyl acid containing groups of the general formula

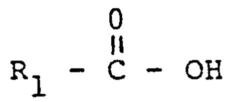

wherein $R_1$ is a hydrogen, linear or branched acylic group, alicylic group, cyclic group, or aryl group and wherein R can in addition, contain one or more functional groups selected from the group consisting of carbonyl, hydroxyl, halogen, ether, nitro, amine, or amide;

f) the phenol promoters contain one or more phenol containing groups of the general formula

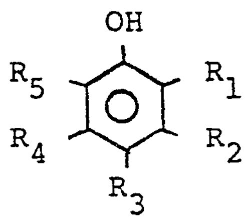

wherein $R_1$, $R_2$, $R_3$, $R_4$, and $R_5$ can be hydrogen, and at least one of $R_1$, $R_2$, $R_3$, $R_4$, and $R_5$ is selected from the group consisting of halogen, hydroxyl, nitro, ether, carbonyl, linear or branched acyclic groups, alicyclic groups, cyclic groups, aryl groups or substituted aryl groups and wherein at least one acyclic or alicyclic group contains at least 11 carbon atoms and wherein in addition the R-designated groups can contain one or more functional groups selected from the group consisting of halogen, ether, nitro, carboxyl, carbonyl, amine, amide, or hydroxyl.

3. A method as described in claim 2 wherein the alkoxylation is carried out using ethylene oxide, propylene oxide, or mixtures of these.

4. A method as described in claim 3 wherein the catalyst is selected from the group consisting of barium metal, barium hydride, barium hydroxide, hydrated barium hydroxide, or barium oxide.

BAD ORIGINAL

5. A method as described in claim 4 wherein the reaction is carried out at a pressure of up to about 100 pounds per square inch gauge.

6. A method as described in claim 5 wherein ethylene oxide is used as the alkoxylation agent and a mole adduct ratio ranges from about 30 weight percent to about 80 weight percent of the ethoxylated product.

7. A method as described in claim 6 wherein the barium containing catalyst is present in an amount of at least about 0.1 by weight based upon the alcohol to be reacted.

8. A method as described in claim 7 wherein the alcohols to be ethoxylated contain from about 4 to about 20 carbon atoms.

9. A method as described in claim 8 wherein the alcohol is a primary alcohol.

10. A method as described in claim 8 wherein the alcohol is the product of a hydroformylation/hydrogenation reaction.

11. A method as described in claim 7 wherein the catalyst promoter is selected from the group consisting of heptanoic acid, ethylene glycol, benzoic acid, benzamide, adipic acid, phenol, stearic acid, bisphenol A (4,4'isopropylidene diphenol), triphenylmethanol, trimethylphenol, hexadecylamine, 1-naphthol, 2-naphthol, azelaic acid, fumaric acid, hydroquinone, salicylic acid, acetophenone, aniline, lauryl aldehyde, benzaldehyde, nonylphenol, o-phenylphenol, benzylamine, citric acid, glycerine, acetylacetone, p-chlorophenol, formamide, 4-methyl-2-pentanone, pyridine, 1,4-butanediol, propylene glycol, p-methoxyphenol, p-nitrophenol, 2-undecanone, 2,2,2-trifluoroethanol, 2-ethylhexanoic acid, acetic acid, mixtures of 2-ethylhexanoic acid and benzamide, or 2-methoxyethanol.

BAD ORIGINAL

12. A method as described in claim 11 when carried out in a continuous reaction.

13. A method as described in claim 2 wherein the promoter is alcohol having a pKa of less than 17.

14. A method as described in claim 13 wherein the alcohol is selected from the group consisting of triphenylmethanol, trichloroethanol, trifluoroethanol, 2-nitroethanol, 2-chloroethanol, 2,2-dichloroethanol, 2-methoxyethanol, 2-chlorocyclohexanol, ortho-chlorobenzyl alcohol.

15. A method for the alkoxylation of alkanols containing from 4 to 36 carbon atoms comprising contacting said alkanols with an alkylating agent selected from the group consisting of ethylene oxide, propylene oxide or mixtures of these in the presence of barium hydride at a temperature of from about 90°C to about 260°C.

16. A method as described in claim 15 wherein barium hydride is promoted with a phenol or substituted phenol of the general formula

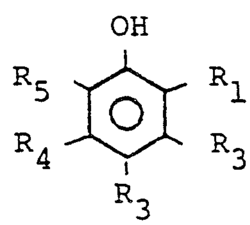

wherein $R_1$, $R_2$, $R_3$, $R_4$, and $R_5$ are, independently, hydrogen or an alkyl group containing from 1 to 10 carbon atoms.

17. A method as described in claim 1 wherein the promoter is a phenol having one or more functional groups of the general formula

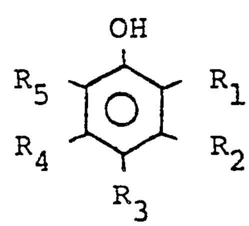

wherein at least one but less than all of $R_1$, $R_2$, $R_3$, $R_4$, and $R_5$ is hydrogen, linear or branched acyclic groups or alicyclic groups having from 1 to 10 carbon atoms and wherein at least one of $R_1$, $R_2$, $R_3$, $R_4$, and $R_5$ is halogen, hydroxyl, nitro, ether, carbonyl, linear or branched acyclic group, alicyclic group, cyclic group, aryl group, or substituted aryl group and wherein in addition the R-designated groups can contain one or more functional groups selected from the group consisting of halogen, ether, nitro, carboxyl, carbonyl, amine, amide or hydroxyl.

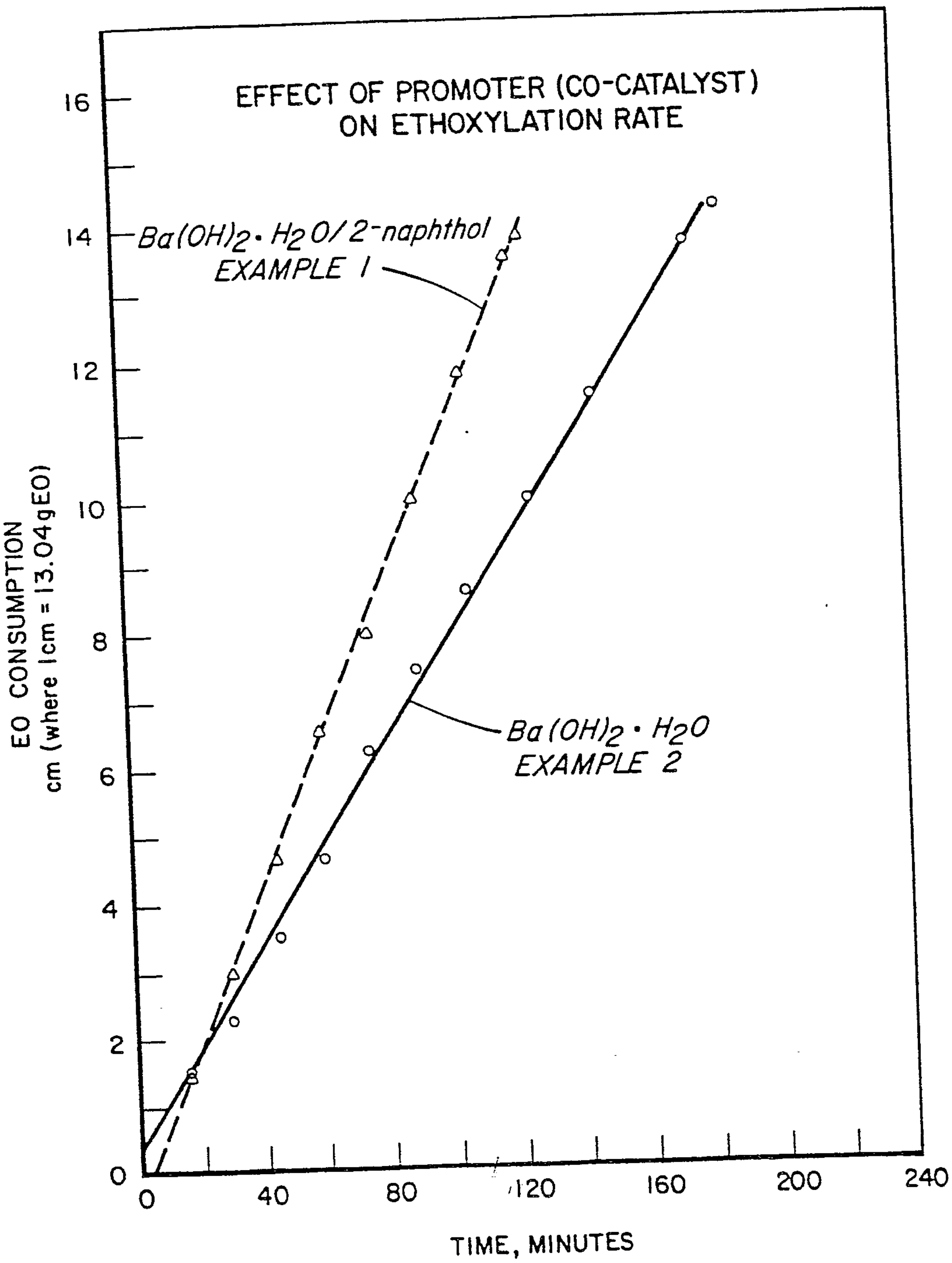
EFFECT OF PROMOTER (CO-CATALYST) ON ETHOXYLATION RATE
Ba(OH)2 · H2O/2-naphthol
EXAMPLE 1
Ba(OH)2 · H2O
EXAMPLE 2
EO CONSUMPTION
cm (where 1cm = 13.04gEO)
TIME, MINUTES
0
2
4
6
8
10
12
14
16
0
40
80
120
160
200
240

Fig. 1

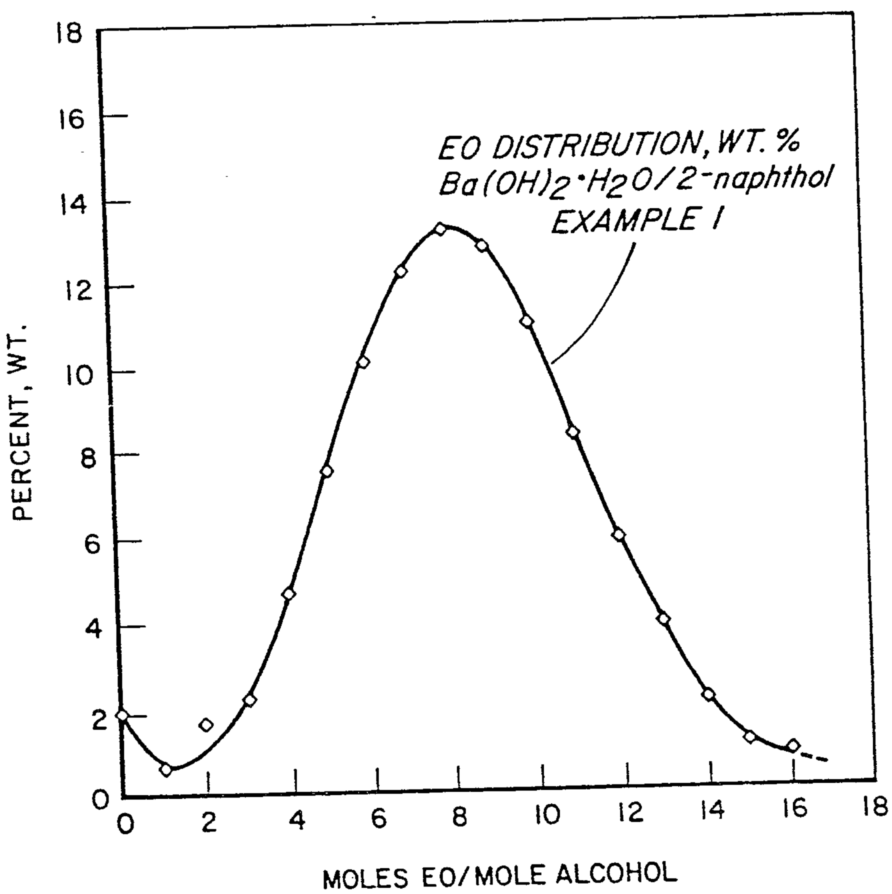
18
16
14
12
10
8
6
4
2
0
PERCENT, WT.
EO DISTRIBUTION, WT. %
Ba(OH)2·H2O/2-naphthol
EXAMPLE I
0
2
4
6
8
10
12
14
16
18
MOLES EO/MOLE ALCOHOL

Fig. 2

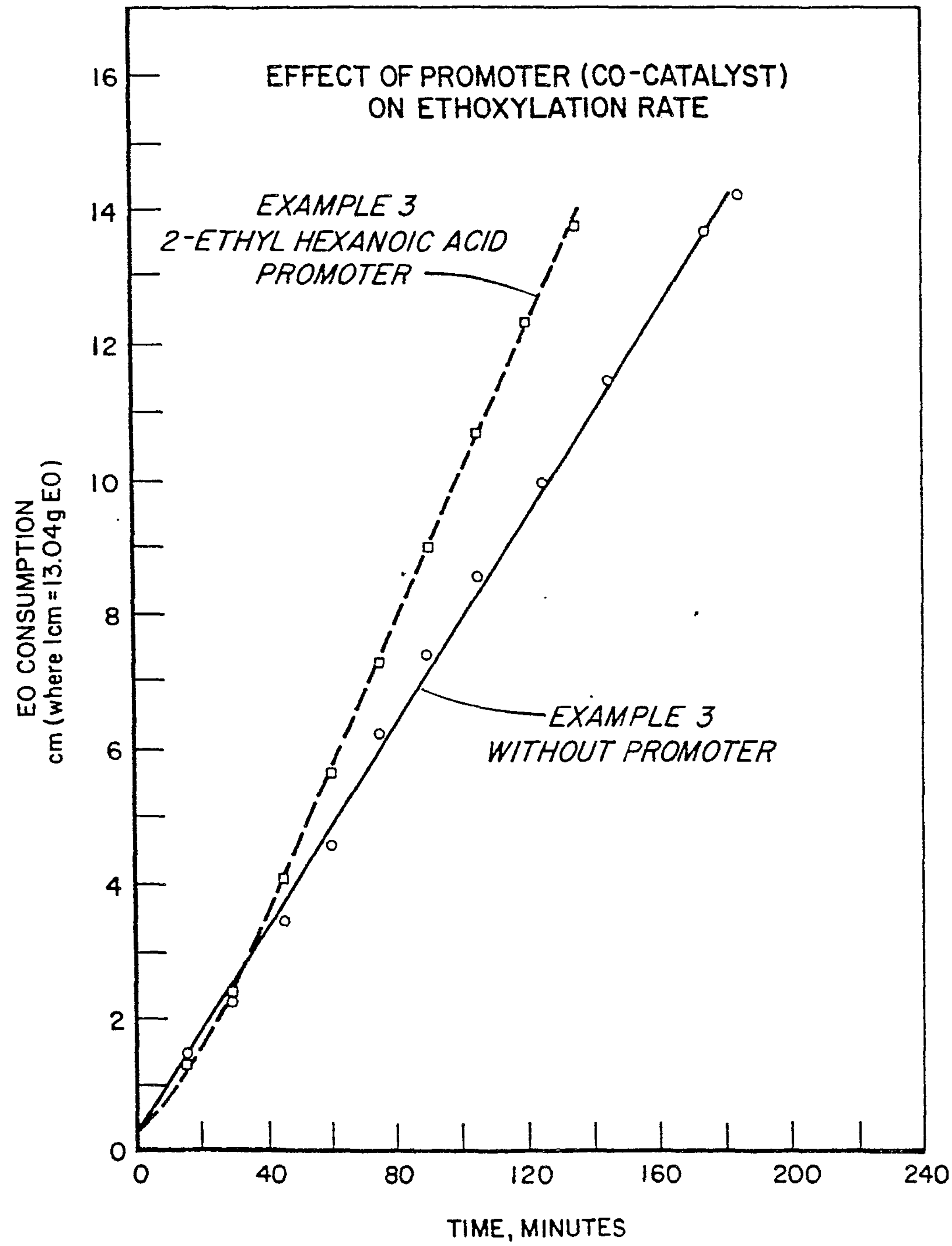
EFFECT OF PROMOTER (CO-CATALYST)
ON ETHOXYLATION RATE
EXAMPLE 3
2-ETHYL HEXANOIC ACID
PROMOTER
EXAMPLE 3
WITHOUT PROMOTER
EO CONSUMPTION
cm (where 1cm = 13.04g EO)
TIME, MINUTES
0
2
4
6
8
10
12
14
16
0
40
80
120
160
200
240

*Fig. 3*

0034650

European Patent Office

# EUROPEAN SEARCH REPORT

Application number

EP 80 10 6316.5

| Category | Citation of document with indication, where appropriate, of relevant passages | Relevant to claim | CLASSIFICATION OF THE APPLICATION (Int. Cl.³) |
|---|---|---|---|
| A | EP - A1 - 0 006 105 (CONTINENTAL OIL) * claim 1 * | 1,3,4 | C 07 C 41/03<br>B 01 J 23/02<br>B 01 J 31/00 |
| E | EP - A1 - 0 020 867 (CONOCO) * claim 1 * | 1 | |
| E | EP - A2 - 0 018 463 (CONOCO) * claim 1 * | 1 | |

DOCUMENTS CONSIDERED TO BE RELEVANT

TECHNICAL FIELDS SEARCHED (Int. Cl.³)

B 01 J 23/02
B 01 J 31/00
B 01 J 31/02
C 07 C 41/01
C 07 C 41/03

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 15-05-1981 | KNAACK |

EPO Form 1503.1 06.78